# EUROPÄISCHE PATENTSCHRIFT

(11) **EP 2 057 940 B1**
(45) Veröffentlichungstag und Bekanntmachung des Hinweises auf die Patenterteilung: **07.12.2011**
(21) Anmeldenummer: 07405322.4
(22) Anmeldetag: 08.11.2007
(51) Int. Cl.: A61B 3/16

(54) **Applanationstonometer**
Applanation tonometer
Tonomètre d'aplanation

(43) Veröffentlichungstag der Anmeldung: 13.05.2009
(73) Patentinhaber: HAAG-STREIT AG, 3098 Köniz (CH)
(72) Erfinder: Siegenthaler, Fritz, 3073 Gümligen (CH)
(74) Vertreter: Rüfenacht, Philipp Michael

(56) Entgegenhaltungen:
- DE-A1- 19 939 348
- FR-A- 2 683 990
- US-A- 5 070 875
- US-A1- 2004 210 123

## Beschreibung

### Technisches Gebiet

Die Erfindung betrifft eine Vorrichtung zur Bestimmung eines Augeninnendruckes eines Auges, mit einer Messanordnung welche einen an einem Messarm befestigten Messkörper zur Applanation des Auges sowie einen auf einer Welle befestigten und um eine Drehachse der Welle drehbaren Drehknopf umfasst, wobei der Messarm radial an einer Schwenkachse befestigt ist, die Messanordnung eine mechanische Kopplung zwischen dem Drehknopf und der Schwenkachse umfasst und eine zur Applanation des Auges benötigte Applanationskraft durch eine Drehung des Drehknopfes um die Drehachse erzeugbar ist. Weiter betrifft die Erfindung ein entsprechendes Verfahren zur Bestimmung eines Augeninnendruckes.

### Stand der Technik

Die Bestimmung des Augeninnendruckes eines Auges eines Patienten ist in der Ophthalmologie eine häufig durchgeführte Untersuchung. Sie dient insbesondere der (Früh-)Erkennung und der Kontrolle bei Glaukom-Erkrankungen (grüner Star). Zur Bestimmung des Augeninnendruckes sind verschiedene Verfahren und entsprechende Geräte bekannt. Es gibt beispielsweise invasive Verfahren, bei welchen entsprechende Drucksensoren direkt in das Auge eingebracht werden. Bei den nicht-invasiven Verfahren kann weiter unterschieden werden zwischen Kontaktmessgeräten, bei denen das Auge bei der Messung berührt wird, und Nicht-Kontakt-Messgeräten, bei denen das Auge bei der Messung nicht direkt berührt wird.

Bei den Nicht-Kontakt-Messgeräten wird das Auge beispielsweise durch einen Luftstrom definierter Stärke applaniert, d. h. abgeplattet. Mit Hilfe einer spezifischen Optik wird hierbei die Geschwindigkeit und das Ausmass der Applanation erfasst und hieraus auf den Augendruck geschlossen.

Zu den nicht-invasiven Verfahren gehört beispielsweise die Bestimmung des Augeninnendruckes mit einem Applanationstonometer nach Goldmann. Bei diesen Geräten wird die Kraft gemessen, die benötigt wird, um einen definierten Bereich, (hier beispielsweise ein kreisflächenförmiger Bereich mit einem Durchmesser von 3.06 mm) des zu untersuchenden Auges zu applanieren, wobei die Applanation typischerweise mit einem Messkörper erfolgt, der eine ebene Applanationsfläche umfasst und bei welchem die aufzubringende Applanationskraft über einen Drehknopf einstellbar ist. Diese Geräte sind dann typischerweise so geeicht, dass der Augeninnendruck direkt an einer Skala am Drehknopf ablesbar ist.

Es gibt unzählige Möglichkeiten, wie bei einem Applanationstonometer die Applanationskraft erzeugt und wie diese dann zum Messkörper übertragen und auf das Auge aufgebracht wird.

Ein Applanationstonometer nach Goldmann ist beispielsweise aus der WO 99/16343 von Heyraud bekannt. Über einen Drehknopf wird ein Gewicht längs der Drehachse des Drehknopfs verschoben, wobei diese Verschiebung mittels einer Mechanik in eine Bewegung der Messsonde in Richtung Auge umgesetzt und auf diese Weise der Applanationsdruck eingestellt wird.

Aus der US 2004/0210123 A1 von J. D. Mueller ist ein Applanationstonometer bekannt, bei welchem durch Drehen eines Einstellknopfes ein Kraftsensor verschoben wird, wobei ein am Kraftsensor positionierter Mitnehmer das untere Ende eines drehbar gelagerten Hebelarms nach hinten (im Gehäuse) drückt, sodass der Messkopf am anderen Ende des Hebelarms auf das Auge gedrückt wird. Der Kraftsensor misst nun, welche Kraft für die Applanation einer bestimmten Fläche notwendig ist, erzeugt ein der gemessenen Applanationskraft entsprechendes Signal und sendet dieses zu einem am Tonometer oder sonstwo positionierten Display, wo die gemessene Applanationskraft bzw. der daraus berechnete Augeninnendruck angezeigt wird.

In Dokument US 5070875 ist ein Applanationstonometer offenbart, welches einen an einem Messarm befestigten Messkörper, eine Schwenkachse mit einem Drehknopf sowie ein auf die Drehachse aufwickelbares Zugübertragungsmittel umfasst, wobei das Zugübertragungsmittel über einen ersten Hebelarm an der Schwenkachse und über einen zweiten Hebelarm an der Drehachse befestigt ist.

Diese Geräte funktionieren zwar einwandfrei, haben jedoch den Nachteil, dass sie einen komplexen Aufbau haben und damit relativ teuer in der Herstellung sind. Zumeist ist auch eine aufwändige und damit wiederum teure Ausbalancierung und vielfach noch eine Kalibrierung der Geräte notwendig.

### Darstellung der Erfindung

Aufgabe der Erfindung ist es, ein dem eingangs genannten technischen Gebiet zugehörende Vorrichtung zur Bestimmung des Augeninnendruckes eines Auges zu schaffen, welche einerseits einfach und damit kostengünstig herzustellen ist, welche andererseits jedoch eine hohe Qualität aufweist und eine präzise Bestimmung des Augeninnendruckes erlaubt. Es ist eine weitere Aufgabe der Erfindung, ein entsprechendes Verfahren zur Bestimmung des Augeninnendruckes eines Auges zu schaffen.

Die Lösung der Aufgabe ist durch die Merkmale des Anspruchs 1 definiert. Die Vorrichtung zur Bestimmung des Augeninnendruckes eines Auges eines Patienten umfasst unter anderem eine Messanordnung, welche wiederum einen an einem Messarm befestigten Messkörper zur Applanation des Auges sowie einen auf einer Welle befestigten und um eine Drehachse der Welle drehbaren Drehknopf umfasst. Der Messarm ist radial an einer Schwenkachse befestigt und die Messanordnung umfasst weiter eine mechanische Kopplung zwischen dem Drehknopf und der Schwenkachse. Die zur Applanation des Auges benötigte Applanationskraft ist durch eine Drehung des Drehknopfes um die Drehachse erzeugbar. Gemäss der Erfindung umfasst die mechanische Kopplung nun Zugübertragungsmittel, welche einerseits via einen ersten Hebelarm an der Schwenkachse und andererseits via einen zweiten Hebelarm an der Drehachse der Welle befestigt sind.

Derartige Geräte, bei welchen der Augeninnendruck durch eine Abplattung der Augenoberfläche, d. h. der Hornhaut, erfolgt, werden typischerweise als Applanationstonometer bezeichnet.

Die Zugübertragungsmittel dienen ausschliesslich zur Übertragung von Zugkräften. D. h. die Kraft, die durch die Drehung der Drehachse und damit auf das an der Drehachse befestigte Ende der Zugübertragungsmittel einwirkt, wird durch die Zugübertragungsmittel an dessen anderes Ende übertragen und übt dort via den ersten Hebelarm ein Drehmoment auf die Schwenkachse aus, welche entsprechend in Drehung versetzt bzw. verschwenkt wird. Bei der Drehung der Drehachse mittels des Drehknopfes werden die Zugübertragungsmittel vorzugsweise auf die Welle aufgewickelt.

Da der Messarm ebenfalls radial an der Schwenkachse befestigt ist, wird so die Drehbewegung des Drehknopfes auf einfache Art und Weise in eine Schwenkbewegung des Messarmes und damit in eine Bewegung des am Messarm befestigen Messkörpers in Richtung Auge umgewandelt. D. h. durch die Zugübertragungsmittel wird eine im Wesentlichen direkte mechanische Kopplung zwischen dem Drehknopf und dem Messarm erreicht, welche im Vergleich zum Stand der Technik äusserst einfach aufgebaut ist. Genau in dieser Einfachheit liegt der grösste Vorteil der Erfindung, denn dadurch lassen sich erfindungsgemässe Applanationstonometer in kostengünstiger Weise herstellen.

Druckkräfte müssen von den Zugübertragungsmitteln hingegen keine übertragen werden können. Entsprechend können die Zugübertragungsmittel aus Materialen bestehen, welche zur Übertragung von Zugkräften geeignet, aber ungeeignet für die Übertragung von Druckkräften sind.

Zudem ist nicht mehr wie beim Stand der Technik eine aufwändige und komplizierte Mechanik notwendig, welche obendrein auch noch mit viel Aufwand und Fingerspitzengefühl ausbalanciert und kalibriert werden muss. Die Kopplung zwischen Drehknopf und Messarm wird durch einfache und damit kostengünstige Mittel erreicht.

Durch die Einfachheit der Konstruktion ist zudem auch gewährleistet, dass sämtliche Komponenten im Gehäuse Platz finden, welches höchstens so gross wie entsprechende, bekannte Tonometergehäuse sind, bzw. das Gerät findet gar in einem kleineren Gehäuse als zuvor Platz. Dies wiederum bedeutet, dass ein Tonometer gemäss der Erfindung derart ausgebildet werden kann, dass es sich äusserlich nicht von einem bekannten Tonometer unterscheidet. Es können mithin beispielsweise also auch dieselben Adapter zur Befestigung des Tonometers an einer Spaltlampe verwendet werden. Selbstverständlich ist hierbei nicht ausgeschlossen, dass das äussere Erscheinungsbild des Tonometers dennoch angepasst werden kann, die mechanischen Schnittstellen für die Befestigung der Adapter werden mit Vorteil jedoch beibehalten.

Bei derartigen Applanationstonometern besteht typischerweise das Problem, dass ihr Nullbereich nicht genau definiert ist, d. h. dass die Stellung des Messarms im Nullbereich bei der Augendruckmessung nicht genau bekannt ist. Sie müssen daher präzise ausbalanciert werden. Dies ist unter anderem notwendig, weil mit einem solchen Tonometer Druckunterschiede von zehntel oder gar hundertstel Millimeter Quecksilbersäule (mmHg) bestimmt werden können sollen.

Bei einer bevorzugten Ausführungsform der Erfindung umfasst das Applanationstonometer zur Ausbalancierung der Messanordnung im Nullbereich daher wenigstens ein Ausgleichsgewicht. Im Stand der Technik wurden zwar auch schon solche Gegengewichte eingesetzt, allerdings waren diese dort derart konstruiert, dass sie zur Ausbalancierung der Messanordnung verschoben oder sonstwie justiert werden konnten und mussten. Demgegenüber sind die Ausgleichsgewichte bei dieser Ausführungsform der Erfindung fixiert und können nach der Montage nicht mehr verschoben, justiert oder sonstwie verändert werden.

Bei einer besonders bevorzugten Ausführungsform der Erfindung ist dieses wenigstens eine Ausgleichsgewicht derart angeordnet und/oder dimensioniert, dass Abstandsänderungen des Auges vom Applanationstonometer im Nullbereich mechanisch kompensiert werden, sodass bei Abstandsänderungen des Auges im Nullbereich ein an der Schwenkachse angreifendes Gesamtdrehmoment als Summe von an der Schwenkachse angreifenden einzelnen Drehmomenten im Wesentlichen konstant ist. Ändert sich der Abstand des Patienten, d. h. des zu untersuchenden Auges vom Tonometer, verschiebt sich nämlich einerseits der Schwerpunkt des wenigstens einen Ausgleichsgewichts und andererseits ändert sich auch die durch die Zugübertragungsmittel auf die Schwenkachse ausgeübte Drehkraft. Ebenso ändert sich die vom Messarm samt Messkörper auf die Schwenkachse ausgeübte Drehkraft.

Bei einer solchen Abstandsänderung ändern sich somit praktisch alle an der Schwenkachse angreifenden Drehmomente. Das wenigstens eine Ausgleichsgewicht ist nun derart angeordnet und/oder dimensioniert, dass eine Summe aller an der Schwenkachse angreifenden Drehmomente im Wesentlichen konstant ist.

Oder mit anderen Worten: Auch wenn der Abstand des Patienten vom Tonometer im Nullbereich variiert, muss - damit durch diese Abstandsänderung kein falscher Augeninnendruck gemessen wird - der Hebeldruck auf das Auge (möglichst) unverändert bleiben. Eine solche Abstandsänderung erzeugt jedoch eine kleine Drehbewegung der Schwenkachse, wodurch der Schwerpunkt der Messoptik sowie der Schwerpunkt des wenigstens einen Ausgleichsgewichts verändert wird und damit ein an der Schwenkachse angreifendes Drehmoment resultiert. Ebenso ändert sich dadurch natürlich auch das von den Zugübertragungsmitteln via den ersten Hebelarm auf die Schwenkachse ausgeübte Drehmoment.

Das wenigstens eine Ausgleichsgewicht ist nun in Grösse und Lage so angeordnet, dass sich bei einer solchen Abstandsänderung von Auge und Tonometer im Nullbereich sämtliche an der Schwenkachse angreifenden Drehmomentänderungen gegenseitig kompensieren, und somit das an der Schwenkachse angreifende Drehmoment und damit auch die vom Messkörper auf das Auge ausgeübte Kraft bei solchen Abstandsänderungen also praktisch unverändert bleibt. Dies gilt wie bereits erwähnt für Abstandsänderungen im Nullbereich, d. h. innerhalb des maximal zulässigen Abstandes des zu messenden Auges vom Messkörper bzw. für den Messabstandsbereich des Tonometers.

Anstelle einer solchen mechanischen Kompensation wäre im Prinzip auch eine elektronische Kompensation dieser Drehmomentänderung möglich. Allerdings müsste hierfür noch wenistens ein zusätzlicher Sensor vorgesehen werden, um die genaue Stellung des Messarms bestimmen zu können. Dies ist jedoch mit zusätzlichem Aufwand verbunden und würde die Herstellung des Tonometers verteuern.

Vorzugsweise sind zwei derartige Ausgleichsgewichte vorgesehen. Ein erstes Ausgleichsgewicht dient als Gegengewicht zum Messarm und ist an der Schwenkachse befestigt und ein zweites Ausgleichsgewicht dient als Gegengewicht zum Messkörper und ist ebenfalls an der Schwenkachse befestigt. Solche Gegengewichte können entweder direkt an der Schwenkachse, z. B. an einer die Schwenkachse bildenden Welle, oder indirekt beispielsweise via einen Hebel an der Schwenkachse befestigt, beispielsweise angeschraubt werden, wobei die gewünschte Befestigungsart z. B. von den Platzverhältnissen innerhalb des Gehäuses des Applanationstonometers abhängt.

Es ist grundsätzlich natürlich auch möglich, eines oder mehrere dieser Ausgleichsgewichte auf mehrere, entsprechend platzierte und dimensionierte Einzelgewichte aufzuteilen. Ebenso ist es möglich, zwei einzelne Ausgleichsgewichte zu einem einzigen, entsprechend schweren und korrekt platzierten Ausgleichsgewicht zusammenzufassen.

Damit die Applanationskraft beispielsweise nicht davon abhängt, mit welcher Geschwindigkeit oder Kraft der Drehknopf gedreht wird, sondern praktisch ausschliesslich von der Winkelstellung des Drehknopfes, umfassen die Zugübertragungsmittel vorzugsweise eine Feder. Dadurch hängt die mittels der Zugübertragungsmittel zum Messarm bzw. zum Messkopf übertragbare Applanationskraft praktisch ausschliesslich von der Auslenkung der Feder aus der Normalposition, d. h. ihrer unbelasteten Position ab und die Auslenkung der Feder wiederum hängt direkt von der Winkelstellung des Drehknopfs ab.

Eine solche Feder kann auf viele verschiedene Arten realisiert werden. So könnten die Zugübertragungsmittel beispielsweise mittels einer Feder an der Drehachse des Drehknopfes befestigt werden. Allerdings besteht hierbei das Problem, dass sich die Federkonstante dieser Feder verändern würde. Wird der Drehknopf gedreht, werden nämlich die an der Drehachse des Drehknopfes befestigten Zugübertragungsmittel und damit diese Feder auf die Welle aufgewickelt, wodurch sich die effektive Federlänge verändern würde. Zudem könnte es sein, dass sich dadurch auch der Hebelarm, via welchen die Feder an der Drehachse befestigt ist, verändert.

Dieselben Probleme bestünden, wenn die Zugübertragungsmittel selber als Feder, beispielsweise als Schraubenfeder, ausgebildet wären.

Auch der Einsatz von Spiral- oder Torsionsfedern wäre im Prinzip möglich. Eine Spiralfeder könnte beispielsweise verwendet werden, um die Zugübertragungsmittel an der Welle oder an einem den ersten Hebelarm bildenden Hebel zu befestigen. Es wäre auch möglich, die Zugübertragungsmittel fix an der Welle zu befestigen und die Welle selber als Torsionsfeder auszubilden oder die Welle ist in zwei koaxiale, nebeneinander liegende, mittels einer Torsionsfeder verbundene Stücke aufgeteilt, wobei die Zugübertragungsmittel an einem und der Drehknopf am andern Stück befestigt wären. Aber auch bei diesen Konstruktionen ergäben sich Probleme, weil typischerweise keine lineare Kraftübertragung erreicht werden kann.

Schliesslich wäre es auch möglich, die Zugübertragungsmittel selber aus einem entsprechenden Material wie z. B. Gummi oder ähnlichen Materialien federnd herzustellen. Allerdings sind die resultierenden Federkonstanten in solchen Fällen auch nicht konstant und die Kraftentfaltung folglich auch nicht linear. Beides ist jedoch von Vorteil, damit die Umrechnung der Applanationskraft in den zu messenden Augeninnendruck möglichst einfach ist.

Bei einer bevorzugten Ausführungsform der Erfindung werden die Zugübertragungsmittel daher mittels einer Feder an einem den ersten Hebelarm bildenden, an der Schwenkachse befestigten Hebel befestigt. Dadurch wird erreicht, dass das andere Ende der Zugübertragungsmittel direkt an der Welle befestigt und somit bei einer Drehung des Drehknopfes um die Drehachse einfach und problemlos auf die Welle aufgewickelt werden kann. Hierbei ist vorzugsweise darauf zu achten, dass die Zugübertragungsmittel nicht mehrlagig auf die Welle aufgewickelt werden. D. h. der Durchmesser der Welle und die auf die Welle aufzuwickelnde Länge der Zugübertragungsmittel sollten entsprechend gewählt werden, weil sich ansonsten wiederum mit der Drehung der Hebelarm an der Drehachse ändern würde.

Die Federkonstante der Feder kann entsprechend dem gewünschten Verhalten gewählt werden. In Frage kommen hierfür mit Vorteil Schraubenfedern. Bei Spiralfedern könnte es wiederum sein, dass sich der Hebelarm ändert, da sich die Feder unter Belastung verformt. Auch eine Schraubenfeder verformt sich natürlich unter Belastung, aber bei solchen Federn ist diese Verformung eindimensional, weshalb sich die effektiven Hebelarme nicht ändern.

Damit die von den Zugübertragungsmitteln übertragene Applanationskraft auch tatsächlich nur von der Auslenkung der Feder abhängt, ist es äusserst wichtig, dass die Zugübertragungsmittel gewisse Eigenschaften aufweisen. Einerseits muss das hierfür verwendete Material biegsam sein, damit es sich auf die Welle aufwickeln lässt und andererseits muss es genügend zugfest sein bzw. darf - bei den zu erwartenden Zugkräften - nur eine sehr geringe oder gar keine Dehnfähigkeit aufweisen, damit die Applanationskraft ohne eine Verformung der Zugübertragungsmittel in Längsrichtung übertragen werden kann.

Zudem hat sich ein Band, d. h. ein langes aber dünnes, also quasi zweidimensionales Gebilde, aus einem solchen Material als vorteilhaft erwiesen, insbesondere im Hinblick auf die regelmässige Aufwickelbarkeit auf die Welle des Drehknopfes. Dies im Gegensatz beispielsweise zu einem eindimensionalen Gebilde wie z. B. einem Faden oder einem Draht bzw. einem dreidimensionalen Gebilde wie z. B. einem Quader oder einem anderen Körper. Bei einem Faden oder Draht mit einer genügend geringen Querschnittsfläche, welche ein problemloses Aufwickeln auf die Welle erlaubt, könnte es nämlich passieren, dass der Faden oder der Draht reisst, wenn via Drehknopf eine hohe Applanationskraft eingestellt wird. Bei einem Quader oder einem anderen dreidimensionalen Körper wiederum ist die Gefahr des Reissens äusserst gering, allerdings ist die Wahrscheinlichkeit hoch, dass der Körper zu wenig flexibel ist, um problemlos und ohne Änderung des Hebelarmes auf die Welle aufgewickelt zu werden.

An sich können die Zugübertragungsmittel aus vielen verschiedenen Materialien hergestellt werden. Die Auswahl reicht beispielsweise von dünnen Folien über Papier bis zu verschiedensten Kunststoffen. Als besonders geeignet haben sich sehr dünne Metallbänder, d. h. Metallbänder mit einer Dicke von weniger als 0.05 mm und einer Breite von einigen Millimetern erwiesen, da sie einen sehr guten Kompromiss zwischen Biegsamkeit und Zugfestigkeit erlauben.

Besonders bevorzugt wird als Zugübertragungsmittel ein dünnes Band aus rostfreiem Stahl verwendet. Dieser ist auf dem Markt zu guten Konditionen erhältlich. Aufgrund der Eigenschaften von Stahl sollte die Dicke des Bandes in diesem Fall 0.02 mm oder weniger betragen.

Damit der Benutzer eines solchen Applanationstonometers den damit gemessenen Augeninnendruck ablesen kann, verfügt das Applanationstonometer vorzugsweise über eine geeignete Anzeigevorrichtung, mit welcher der der Applanationskraft entsprechende Drehwinkel des Drehknopfes optisch darstellbar ist. Anstatt oder zusätzlich zu einer optischen Darstellung, wäre es auch möglich, den gemessenen Druck akustisch, beispielsweise über einen Lautsprecher, auszugeben oder ihn beispielsweise auf Papier auszudrucken.

Die Anzeigevorrichtung kann hierbei wie aus dem Stand der Technik bekannt eine auf dem Drehknopf aufgebrachte Skala mit einem auf dem Gehäuse positionierten Referenzpunkt (oder umgekehrt) beinhalten. Sie könnte beispielsweise auch in der Art der Datumsanzeige bei (mechanischen) Armbanduhren, bei welchen eine Scheibe mit einer darauf aufgebrachten Skala unter einer entsprechenden Öffnung, dem Datumsfenster, vorbeigedreht wird, oder anderweitig mit einer entsprechenden Mechanik realisiert sein.

Vorzugsweise umfasst die Anzeigevorrichtung allerdings ein Display, auf welchem die Applanationskraft anzeigbar ist. Ein solches Display könnte beispielsweise als 7-Segment Anzeige, als Liquid Crystal Display (LCD) oder auch mittels Leuchtdioden oder anderen derartigen Displays realisiert sein. Gegebenenfalls müsste die via den Drehwinkel mit dem Drehknopf eingestellte Applanationskraft noch in ein entsprechendes elektrisches Signal umgewandelt werden.

Um die Handhabung und die Bedienung des Geräts zu vereinfachen, wird das Applanationstonometer vorzugsweise mit einem Mikroprozessor und einem an der Welle befestigten Winkelgeber ausgestattet.

Der Winkelgeber dient hierbei zur Umwandlung des eingestellten, der Applanationskraft entsprechenden Drehwinkels in ein elektrisches Signal. Dieses ist dann vom Mikroprozessor in einen die Applanationskraft repräsentierenden, digitalen Wert umwandelbar. Dieser digitale Wert kann dann direkt mit der Anzeigevorrichtung optisch dargestellt werden.

Damit einerseits die Umwandlung des Drehwinkels in einen digitalen Wert möglichst einfach wird und damit andererseits eine möglichst einfache Eichung bzw. Kalibrierung des Winkelgebers möglich ist, sollte dieser möglichst linear sein. D. h. zwischen dem Drehwinkel und dem vom Winkelgeber gelieferten elektrischen Signal besteht eine lineare Abhängigkeit. In diesem Fall wird die Kennlinie des Winkelgebers nämlich durch eine Gerade gebildet, wobei zwei Kalibrierungspunkte ausreichen, um die Steigung dieser Geraden zu bestimmen, welche für die Umrechnung des Drehwinkels in die tatsächlich aufgebrachte Applanationskraft benötigt wird.

Zwar könnte auch ein nicht linearer Winkelgeber eingesetzt werden, aber dadurch steigt nicht nur der Aufwand für die Umrechnung selber, auch die Anzahl der notwendigen Kalibrierungspunkte zur Bestimmung der Kennlinie des Winkelgebers muss unter Umständen massiv erhöht werden.

Anstatt oder zusätzlich zur optischen Anzeige des gemessenen Augeninnendruckes, kann dieser beispielsweise mittels einer Funkübertragungstechnik an einen entsprechenden Empfänger übertragen werden. Ein solcher Empfänger kann beispielsweise ein anderes Gerät wie etwa ein Computer, ein Mobilfunktelefon, ein PDA (personal digital assistant) oder ein anderes geeignetes Gerät sein. Das Applanationstonometer wird mit einem entsprechenden Sender und allenfalls einem entsprechenden Empfänger ausgerüstet. Es wäre im Prinzip zwar möglich, hierfür eine drahtlose Übertragungstechnologie zu verwenden, mit welchem Signale auch über grosse Distanzen übertragen werden können. Beispielsweise könnte eine Übertragungstechnologie eingesetzt werden, wie sie auch bei Mobilfunktelefonen (z. B. GSM, UMTS oder ähnliche) verwendet wird. Da die Übertragung typischerweise jedoch an ein Gerät erfolgen wird, welches sich in unmittelbarer Nähe, d. h. in einer Distanz von typischerweise höchstens einigen Metern vom Applanationstonometer befindet, wird das Applanationstonometer vorzugsweise mit einem Sender und gegebenenfalls einem Empfänger für eine kurzreichweitige Funkübertragungstechnik ausgestattet.

Bluetooth ist ein Beispiel für eine solche kurzreichweitige Funkübertragungstechnologie, wobei entsprechende Bluetooth Sender/Empfänger als integrierte Bauteile, d. h. als Chip, äusserst kostengünstig erhältlich sind und daher bevorzugt eingesetzt werden.

Wie bereits vorgängig beschrieben worden ist, können Applanationstonometer auf verschiedene Weise realisiert werden, wobei aufgrund ihrer einfachen Handhabbarkeit, ihrer Genauigkeit und ihrer weiten Verbreitung häufig Applanationstonometer nach Goldmann eingesetzt werden. Bei solchen Geräten wird eine kreisförmige Fläche mit einem Durchmesser von 3.06 mm applaniert, d. h. abgeplattet. Da in diesem Fall der Augeninnendruck (in mmHg) genau dem 10-fachen der für die Applanation notwendigen Applanationskraft (in Gramm gemessen) entspricht, ist das Applanationstonometer gemäss der Erfindung vorzugsweise als Applanationstonometer nach Goldmann ausgebildet.

Die Lösung der Aufgabe bezüglich dem Verfahren zur Bestimmung des Augeninnendruckes eines Auges ist durch die Merkmale des Anspruchs 12 definiert. Hierbei wird das Auge mit einem Messkörper, welcher an einem radial an einer Schwenkachse befestigten Messarm befestigt ist, applaniert, wobei die zur Applanation benötigte Applanationskraft durch eine Drehung eines auf einer Welle befestigten Drehknopfes um eine Drehachse erzeugt und mittels einer mechanischen Kopplung zwischen dem Drehknopf und der Schwenkachse auf den Messarm übertragen wird. Erfindungsgemäss wird hierbei die Applanationskraft mittels eines Zugübertragungsmittels auf den Messarm übertragen, indem die Zugübertragungsmittel einerseits via einen ersten Hebelarm an der Schwenkachse und andererseits via einen zweiten Hebelarm an der Drehachse der Welle befestigt werden.

Das Verfahren zur Bestimmung des Augeninnendruckes mit einem erfindungsgemässen Applanationstonometer umfasst weiter vorzugsweise folgende Schritte:

Das Applanationstonometer wird typischerweise derart auf einer Spaltlampe montiert, dass dessen Benutzer durch das Mikroskop der Spaltlampe sowie den Messkörper des Applanationstonometers hindurch das Auge betrachten kann, dessen Innendruck bestimmt werden soll. Der Messkörper ist entsprechend zumindest teil-transparent ausgebildet. Als Nächstes wird der Patient, typischerweise mit Hilfe einer Halterung wie beispielsweise einer Kinnstütze derart vor der Spaltlampe positioniert, dass das zu untersuchende Auge durch das Mikroskop und den Messkörper hindurch betrachtbar ist. Dann wird das Applanationstonometer leicht vorgespannt, sodass sich der Messarm quasi in der Nullposition befindet. Der Benutzer fährt nun mit der Spaltlampe und dem davor fixierten Applanationstonometer zum Auge hin, bis der Messkörper das Auge möglichst zentral ganz leicht berührt. Direkt vor der Messung erfolgt typischerweise eine normale Anästhesie des zu untersuchenden bzw. beider Augen des Patienten. Zusätzlich sollte zur besseren Visualisierung der Applanation auch noch ein Fluorescein auf das Auge gegeben werden. Danach wird die Position des Applanationstonometers nicht mehr wesentlich verändert, sondern über die Drehung des Drehknopfes wird die Applanationskraft erhöht, bis die gewünschte Hornhautfläche applaniert ist. Bei einem Applanationstonometer nach Goldmann ist dies eine kreisförmige Fläche mit einem Durchmesser von 3.06 mm. Selbstverständlich kann die Position des Messkörpers auf dem Auge auch während einer Messung noch korrigiert werden.

Daraufhin kann die Spaltlampe mit dem Applanationstonometer vom Auge weggefahren und der Augeninnendruck am Applanationstonometer abgelesen werden. Beispielsweise wie bereits erwähnt an einer auf dem Drehknopf oder am Gehäuse aufgebrachten Skala oder an einem am Gehäuse des Applanationstonometers angebrachten Display.

Wie beschrieben kann der auf diese Weise ermittelte Augeninnendruck in Form eines digitalen Wertes auch mittels eines Senders per Funk an einen entsprechenden Empfänger übermittelt werden. Ein solcher Empfänger kann beispielsweise in einen Computer, ein Mobiltelefon, einen PDA oder ein beliebiges anderes Gerät integriert sein. Der übermittelte Augeninnendruck kann dann beispielsweise in dem Gerät abgespeichert oder an einer Anzeigevorrichtung dieses Geräts angezeigt werden. Typischerweise werden zusammen mit dem gemessenen Augeninnendruck auch noch anderen Angaben abgespeichert: Der Name und weitere persönliche Daten wie z. B. das Geburtsdatum des Patienten, das Datum der Messung und ob es sich um den Innendruck des linken oder des rechten Auges (links/rechts - Erkennung) handelt. Einige dieser Daten wie z. B. der Name und die persönlichen Angaben zum Patienten sind beim Empfänger unter Umständen schon bekannt oder können dort vom Benutzer eingegeben werden. Andere Angaben wie z. B. die links/rechts Angaben über das gemessene Auge können während der Messung vom Applanationstonometer selber bestimmt und zusammen mit dem ermittelten Augeninnendruck an das Gerät übermittelt werden.

Für die links/rechts Erkennung verfügt das Applanationstonometer vorzugsweise über einen entsprechenden Sensor, beispielsweise einen Hall-Sensor. Dieser kann anhand eines in der Kinnstütze der Spaltlampe befestigten Magneten bestimmen, ob der Innendruck des linken oder des rechten Auges bestimmt wird.

Die Übertragung dieser Angaben vom Applanationstonometer an ein externes Gerät kann hierbei sowohl automatisch nach Abschluss der Messung oder bevorzugt manuell durch Drücken eines entsprechendes Knopfes am Applanationstonometer erfolgen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine schematische Seitenansicht des Innenlebens eines Applanationstono- meters gemäss der Erfindung;
- Fig. 2: eine schematische Rückansicht des Applanationstonometers aus Fig. 1;
- Fig. 3: eine schematische Seitenansicht des Applanationstonometers aus Fig. 1 während einer Messung mit einer hohen eingestellten Applanationskraft;
- Fig. 4: eine schematische Rückansicht des Applanationstonometers aus Fig. 3;
- Fig. 5: eine schematische Darstellung der Befestigung der Zugübertragungsmittel an der Schwenkachse;
- Fig. 6: eine schematische Seitenansicht des Innenlebens einer zweiten Ausführungsform eines Applanationstonometers gemäss der Erfindung;
- Fig. 7: eine schematische Rückansicht des Applanationstonometers aus Fig. 6
- Fig. 8: eine schematische Vorderansicht des Applanationstonometers aus Fig. 6.

Grundsätzlich sind in den Figuren gleiche Teile mit gleichen Bezugszeichen versehen.

### Wege zur Ausführung der Erfindung

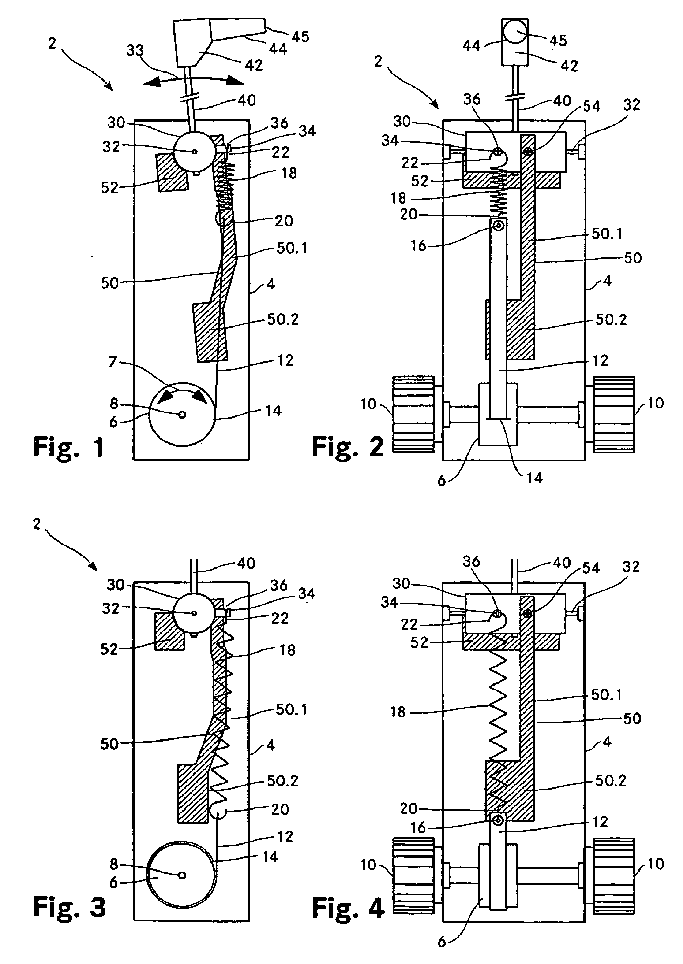

Die Figuren 1 und 2 zeigen eine schematische Darstellung eines Applanationstonometers 2 gemäss der Erfindung. In Figur 1 ist das Innenleben des Applanationstonometers 2 in einer Seitenansicht und in Figur 2 in einer Rückansicht dargestellt. Was Vorder- und Rückseite des Applanationstonometers 2 ist, wird hierbei aus der Sicht des Benutzers, der den Innendruck eines Auges eines Patienten bestimmt, definiert. D.h. die Rückansicht ist die Ansicht jener Seite des Applanationstonometers 2, welche während der Messung dem Patienten zugewandt ist.

Das Applanationstonometer 2 umfasst ein Gehäuse 4, wobei im unteren Bereich des Gehäuses 4 eine Welle 6 positioniert ist, welche um ihre Drehachse 8 drehbar (angedeutet durch den Pfeil 7) gelagert ist. Auf derselben Drehachse ist ausserhalb des Gehäuses 4 auf wenigstens einer Seite des Gehäuses 4, vorzugsweise wie dargestellt jedoch auf beiden Seiten des Gehäuses 4, ein Drehknopf 10 positioniert. An dieser Welle ist das untere Ende eines Zugübertragungsmittels, in diesem Fall eines dünnen Stahlbands 12 befestigt, hier beispielsweise in eine Öffnung 14 in der Welle 6 eingeklebt. Das Stahlband 12 ist in diesem Beispiel 0.02 mm dünn und einige Millimeter breit. Das obere Ende des Stahlbandes 12 umfasst eine kleine Öffnung 16, beispielsweise eine Öse, in welche das untere Ende einer Schraubenfeder 18, d. h. das zu einer Öse 20 gebogene, untere Ende des Drahtes der Schraubenfeder 18, eingehängt ist.

Im oberen Bereich des Gehäuses 4 ist eine weitere Welle 30 positioniert, welche um ihre Schwenkachse 32 drehbar gelagert ist (angedeutet durch den Pfeil 33), wobei die Schwenkachse 32 parallel zur Drehachse 8 ausgerichtet ist. An dieser Welle 30 ist radial ein Hebel 34 angebracht, welcher eine kleine Ausnehmung 36 umfasst, in welche das obere Ende der Schraubenfeder 18, welches ebenfalls zu einer Öse 22 gebogen ist, eingehängt ist. Die beiden Ösen 20, 22 der Schraubenfeder 18 sind hierbei senkrecht zueinander ausgerichtet, d. h. die durch die Ösen 20, 22 definierten Ebenen stehen im Wesentlichen im rechten Winkel zueinander.

An der Welle 30 ist weiter ebenfalls radial ein Messarm 40 befestigt, welcher an seinem oberen Ende eine Halterung 42 aufweist, in welcher der Messkörper 44 befestigt werden kann. Der Messkörper selber entspricht den handelsüblichen Messkörpern und ist entsprechend aus einem transparenten Material gefertigt. Zur Applanation des Auges umfasst er eine kreisflächenförmige Applanationsfläche 45 mit einem Durchmesser von 3.06 mm gemäss dem Goldmann Standard. Wird nun unmittelbar vor der Messung ein fluoreszierender Farbstoff (z. B. Fluoreszin) ins Auge gegeben und das Auge während der Messung mit Blaulicht beleuchtet, leuchtet am Rand jenes Bereichs der Applanationsfläche 45, in welchem der Messkörper das Fluoreszin verdrängt, ein Farbring auf. Wie bei Goldmann-Tonometern üblich, umfasst der Messkörper weiter ein Prisma, welches bei der Durchsicht durch den Messkörper hindurch die obere Bildhälfte gegen die untere Bildhälfte verschiebt, wobei die Verschiebung genau dem gewünschten Durchmesser der abzuflachenden Fläche entspricht. Anhand der Position der beiden sichtbaren Halbkreise kann der Benutzer folglich erkennen, ob die Augenoberfläche zu wenig, korrekt oder zu viel applaniert ist und entsprechend die Applanationskraft durch Drehen am Drehknopf erhöhen, unverändert belassen oder verringern.

Zur Auswuchtung der Messanordung mit Welle 6, Stahlband 12, Schraubenfeder 18, Hebel 34, Welle 30 und Messarm 40 mit Messkörper 44 sind zwei Ausgleichsgewichte 50, 52 an der Welle 30 befestigt. Das erste Ausgleichsgewicht 50 dient hierbei quasi als Gegengewicht zum Messarm 40 und umfasst einen mittels einer Schraube 54 an der Welle 30 befestigten Bügel 50.1 und ein am unteren Ende des Bügels befestigtes Gewicht 50.2. Selbstverständlich ist es auch möglich, Bügel 50.1 und Gewicht 50.2 einstückig auszubilden. Das zweite Ausgleichsgewicht 52 dient als Gegengewicht zum Messkörper 44 und ist mittels einer Schraube (nicht sichtbar) direkt an der dem Ausgleichsgewicht 50 gegenüberliegenden Seite der Welle 30 befestigt.

Diese Aufteilung der notwendigen Gegengewichte zur Ausbalancierung der Messanordnung ist nicht zwingend notwendig. Wenn es die Platzverhältnisse innerhalb des Gehäuses 4 zulassen, kann selbstverständlich auch nur ein einziges, entsprechend schweres und korrekt positioniertes Gegengewicht an der Welle 30 befestigt werden. Der Vollständigkeit halber sei erwähnt, dass auch eine Aufteilung in mehr als zwei einzelne Gegengewichte möglich wäre.

Ist die Schraubenfeder 18 entspannt, d. h. die Welle 6 gemäss Darstellung in Fig. 1 entsprechend weit im Gegenuhrzeigersinn gedreht, ist die Messanordnung derart ausbalanciert, dass der Messarm 40 samt Messkopf 44 seine extremste Auslenkung nach links (d. h. ebenfalls im Gegenuhrzeigersinn) erfährt. Diese Position des Messarms 40 ist in Fig. 1 dargestellt. Während der Messung wird nun die Welle 6 per Drehknopf 10 soweit im Uhrzeigersinn gedreht, bis der Messarm 40 in seiner Nullposition ist, d. h. in etwa senkrecht steht, womit die Schraubenfeder 18 leicht vorgespannt ist. In dieser Position wird das gesamte Applanationstonometer 2 vor das Auge geführt, bis der Messkopf 44 das zu messende Auge bzw. dessen Hornhaut leicht berührt. Danach wird der auf das Auge ausgeübte Applanationsdruck erhöht, indem die Welle 6 im Uhrzeigersinn weitergedreht wird. Dadurch wird das Stahlband 12 auf die Welle 6 aufgewickelt und die Schraubenfeder 18 wird immer weiter gespannt. Dies ist in den Figuren 3 (Seitenansicht) und 4 (Rückansicht) dargestellt. Es ist zu beachten, dass die Länge des auf die Welle 6 aufgewickelten Stahlbandes 12 derart bemessen ist, dass - wie aus Fig. 3 ersichtlich, das Stahlband 12 nicht mehrlagig auf die Welle 6 aufgewickelt wird. D. h. diese Länge darf bei der grössten erzeugbaren Applanationskraft höchstens D * π betragen (wobei D den Durchmesser der Welle 6 und π die Kreiszahl bezeichnet), da sich ansonsten der effektive Hebelarm und damit das an der Drehachse 8 wirkende Drehmoment verändert, was wiederum einen Einfluss auf die Präzision der Augeninnendruck-Messung haben kann.

Da der Messkopf 44 bereits auf dem Auge aufliegt, wird die Winkelstellung des Messarms 40 beim Weiterdrehen der Welle nur noch ganz leicht im Uhrzeigersinn weitergedreht, wohingegen sich die Applanationskraft und die vom Messkopf 44 applanierte Fläche der Hornhaut vergrössert. Ist die gewünschte Fläche applaniert, kann aus der Winkelstellung der Drehknöpfe die Applanationskraft berechnet werden, wobei das Applanationstonometer 2 hierfür vom Auge weggenommen werden kann.

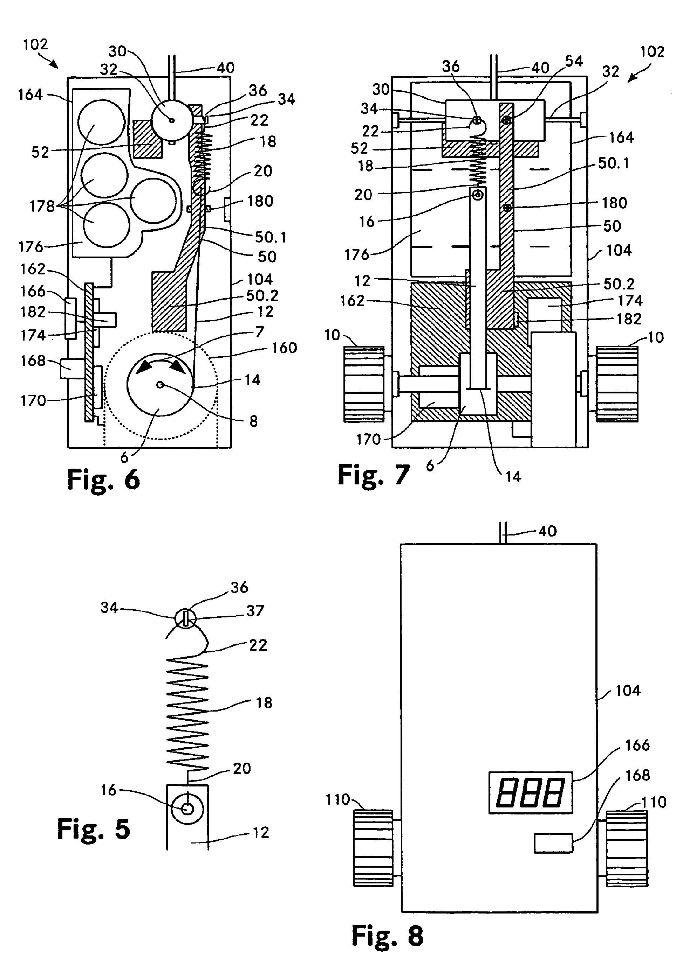

Das Ablesen der aufgebrachten Applanationskraft erfolgt entweder an einer Skala, welche auf einem oder beiden Drehknöpfen bzw. am Gehäuse 4 aufgebracht und derart geeicht ist, dass - wie bei Applanationstonometern nach Goldmann üblich - an der Skala direkt die eingestellte Applanationskraft abgelesen werden kann. Oder aus der mittels der Drehknöpfe eingestellten Drehwinkel wird - wie in dem in den Figuren 6 bis 8 dargestellten und weiter unten beschriebenen Beispiel - ein der Applanationskraft entsprechender, digitaler Wert erzeugt, welcher an einem Display am Gehäuse 4 als Applanationskraft angezeigt oder per Funk an ein anderes Gerät übermittelt und dort weiterverarbeitet wird.

Um die Übertragung der Zugkräfte zwischen der Schraubenfeder 18 und dem Stahlband 12 einerseits sowie dem Hebel 36 andererseits möglichst kontrollieren zu können und so reproduzierbar wie möglich zu gestalten, sollte verhindert werden, dass sich der Berührungspunkt zwischen Feder und Stahlband 12 bzw. Hebel 36 nach der Kalibrierung des Geräts wieder verändern kann. Hierfür wird in wahlweise eine oder beide Ösen der Schraubenfeder 18 ein Knick 37 eingebracht. Dadurch kann sich der Kraftangriffspunkt nicht mehr verändern.

Dieser Knick wird vorzugsweise derart angebracht, dass er möglichst genau auf der Längsachse der Schraubenfeder 18 liegt, damit die Richtung der auf die Schraubenfeder 18 wirkenden Kraft möglichst genau mit der Richtung der Längsachse der Schraubenfeder übereinstimmt. Wenn der Knick nicht auf dieser Längsachse liegt, ergibt sich zwischen Richtung der wirkenden Kraft und der Richtung der Längsachse ein Winkel verschieden von Null, was unter Umständen zu verfälschten Messungen führen könnte.

In den Figuren 6 und 7 ist ein weiteres Ausführungsbeispiel eines Applanationstonometers 102 gemäss der Erfindung dargestellt. Mechanisch ist dieses Gerät praktisch identisch mit dem in den Figuren 1 bis 4 dargestellten Applanationstonometer 2. Der Hauptunterschied besteht darin, dass das Applanationstonometer 102 über eine eigene Stromversorgung verfügt und der gemessene, dem Augeninnendruck entsprechende Applanationsdruck in ein elektrisches Signal umgewandelt und an einem Display dargestellt bzw. per Funk an ein externes Gerät übertragen werden kann.

Hierfür umfasst das Applanationstonometer 102 einige weitere Bauteile: So unter anderem einen Winkelgeber 160, eine mit diversen Bauteilen bestückte Leiterplatte 162, eine Stromversorgung 164, ein Display 166, sowie einen Einschaltknopf 168. Bei dem Winkelgeber 160 handelt es sich um ein elektromechanisches Bauteil, welches an die Welle 6 angekuppelt ist und die Winkelposition der Welle 6 in elektrisch auswertbare, gegebenenfalls kodierte Daten, typischerweise in ein elektrisches Signal, umsetzt. Dieses Signal wird an einen auf der Leiterplatte 162 bestückten Mikroprozessor 170 weitergeleitet, wofür der Winkelgeber 160 elektrisch mit der Leiterplatte 162 verbunden ist.

Der Mikroprozessor 170 wandelt das vom Winkelgeber 160 stammende, elektrische Signal in einen der Applanationskraft und damit dem Augeninnendruck entsprechenden digitalen Wert um und sendet diesen an das Display 166, auf welchem der digitale Wert angezeigt wird. Damit der Mikroprozessor zu einer bestimmten Winkelstellung der Welle 6 den korrekten Augeninnendruck ausgeben und auf dem Display anzeigen kann, muss er zuvor jedoch entsprechend geeicht werden. Hierfür muss die Kennlinie der Messanordnung bestimmt werden, d. h. der Zusammenhang zwischen der Winkelstellung der Welle 6 und der vom Messkörper auf das Auge ausgeübten Applanationskraft.

Da ein linearer Winkelgeber 160 wie auch eine Schraubenfeder 18 mit linearer Kraftentfaltung verwendet wird, ist die Kennlinie der Messanordnung eine Gerade und es reichen zwei Punkte dieser Kennlinie, um diese zu bestimmen. Zur Eichung des Geräts wird deshalb solange am Drehknopf gedreht, bis vom Messkopf eine bestimmter Druck auf ein Testobjekt ausgeübt wird, wobei dieser Druck mit einem entsprechend geeichten Druckmessgerät gemessen wird. Diese Prozedur wird dann für einen zweiten Druckwert wiederholt und der Mikroprozessor wird so programmiert, dass er bei diesen Winkelstellungen der Welle 6 den jeweils eingestellten Druck als Augeninnendruck auf dem Display ausgibt. Zwischen diesen beiden Punkten und darüber hinaus kann dann die Kennlinie inter- bzw. extrapoliert werden, sodass der Mikroprozessor für jede Winkelstellung der Welle 6 den korrekten Applanationsdruck berechnen und auf dem Display ausgeben kann. Sicherheitshalber können auf diese Weise auch drei oder mehr Punkte der Kennlinie bestimmt werden, sodass die Kennlinie beispielsweise durch mehrere Geradenabschnitte angenähert werden kann. Um den Aufwand zu minimieren, sollte die Anzahl der notwendigen Eichpunkte jedoch möglichst reduziert werden.

Grundsätzlich funktioniert das Applanationstonometer natürlich auch mit einem nicht-linearen Winkelgeber und einer nicht-linearen Schraubenfeder. Allerdings ist der Aufwand zur Eichung der Umrechnung der Winkelstellung der Welle 6 in den gemessenen Augeninnendruck erheblich höher, da die Kennlinie des Systems durch eine Vielzahl von Eichpunkten bestimmt werden muss. Auch die Umrechnung durch den Mikroprozessor ist in diesem Fall typischerweise erheblich höher.

Das Applanationstonometer 102 verfügt des Weiteren über einen Bluetooth-Chip 174, welcher ebenfalls auf der Leiterplatte 162 bestückt ist und mittels welchem der vom Mikroprozessor 170 berechnete Augeninnendruck per Bluetooth-Technologie an einen entsprechenden Bluetooth-Empfänger (nicht dargestellt) gesendet werden kann. Dieser Bluetooth-Empfänger kann beispielsweise in einen Comuter, ein Mobiltelefon, einen PDA oder ein anderes, hierfür geeignetes Gerät integriert oder daran angeschlossen sein. Der gemessene und übertragene Augeninnendruck kann beim empfangenden Gerät dann beliebig weiterverarbeitet, beispielsweise an einem dort angeschlossenen Display angezeigt oder in einem Speicher abgespeichert werden.

Das Übermitteln des gemessenen Augeninnendruckes (und allenfalls weiterer Angaben wie z. B. Angaben darüber, ob es sich um das linke oder das rechte Auge des Patienten handelt), erfolgt bei eingeschaltetem Applanationstonometer 102 durch Drücken des Einschaltknopfs 168. Um das Gerät abzuschalten, muss hingegen weder dieser noch ein anderer Knopf gedrückt werden, denn das Gerät schaltet sich bei Inaktivität nach einer vordefinierten Zeit, typischerweise nach einigen Sekunden, selber aus.

Die benötigte elektrische Energie wird hierbei von der Stromversorgung 164 geliefert. Diese umfasst beispielsweise eine Mehrzahl von in einem Batteriegehäuse 176 untergebrachten Batterien 178, wobei vorzugsweise handelsübliche Batterien verwendet werden können. Grundsätzlich wäre es aber natürlich auch möglich, andere Energiequellen wie etwa Solarzellen zu verwenden oder das Applanationstonometer 102 per Kabel an eine externe Energiequelle anzuschliessen.

Um die Sicherheit des Patienten zu gewährleisten, d. h. Verletzungen des Auges, dessen Innendruck bestimmt wird, zu verhindern, sind diverse Sicherheitsmassnahmen vorgesehen. Einerseits verfügt das Applanationstonometer 102 über mechanische Anschläge, die den Ausschlag des Messarms 40 in beide Richtungen begrenzen. Hierfür ist beispielsweise eine verstellbare Schraube, beispielsweise eine Madenschraube 180 mit vorgegebener Länge in den Bügel 50.1 des Ausgleichsgewichts eingelassen. Diese schlägt einerseits an dem Batteriegehäuse 176 und andererseits an der Innenseite des Gehäuses 104 an. Die Länge der Madenschraube 180 ist hierbei derat bemessen, dass sich der Messkopf 44 ca. 9 mm hin- und herbewegen kann. Von der Nullposition aus sind es ca. 2.5 mm in Richtung des Auges und ca. 6.5 mm in Richtung vom Auge weg. Der Bereich, den sich der Messkopf 44 von der Nullposition aus in Richtung Auge bewegen kann - im vorliegenden Beispiel sind es rund 2.5 mm - wird als Nullbereich bezeichnet. Dieser Nullbereich entspricht dem maximal zulässigen Abstand des Auges vom Messkörper 44 und wird auch als Messabstandsbereich des Tonometers bezeichnet.

Die mechanischen Anschläge können selbstverständlich auch auf andere Art und Weise realisiert werden, beispielsweise durch entsprechend positionierte Bauteile, welche entsprechend die Auslenkung des Messarms 40 beschränken.

Andererseits wird die Auslenkung des Messarms 40 überwacht. Hierfür ist auf der Leiterplatte 162 ein Sensor 182 bestückt, welcher die Distanz des Gewichts 50.2 von der Leiterplatte 162 bestimmt und laufend an den Mikroprozessor 170 übermittelt. Der Mikroprozessor 170 gibt dann einen Alarm aus, beispielsweise in Form eines akustischen Signals via einen Lautsprecher (nicht dargestellt), wenn diese Distanz ausserhalb eines erlaubten Bereichs liegt. Es wird insbesondere dann ein Alarmsignal ausgegeben, wenn die Distanz zu gross wird. Dadurch kann verhindert werden, dass der Benutzer des Geräts das Auge verletzt, indem er das Applanationstonometer 102 mit einem bereits am Anschlag anliegenden Messarm 40 samt Messkörper 44 unbeabsichtigt weiter zum Auge hin bewegt.

Fig. 8 schliesslich zeigt eine schematische Vorderansicht des Gehäuses 104 des Applanationstonometers 102 aus den Fig. 6 und 7. Neben dem Gehäuse 104, den Drehknöpfen 110 und dem Messarm 40 sind das Display 166 sowie der Einschaltknopf 168 dargestellt.

Zusammenfassend ist festzustellen, dass mit der Erfindung ein sehr einfach aufgebautes Applanationstonometer geschaffen wurde, welches in der Herstellung äusserst kostengünstig ist, bei welchem jedoch keine Abstriche betreffend Qualität sowie Messgenauigkeit und -reproduzierbarkeit gemacht werden müssen.

## Patentansprüche

1. Vorrichtung zur Bestimmung eines Augenlnnendruckes eines Auges, mit einer Messanordnung, welche einen an einem Messarm (40) befestigten Messkörper (44) zur Applanation des Auges sowie einen auf einer Welle (6) befestigten und um eine Drehachse (8) der Welle (6) drehbaren Drehknopf (10) umfasst, wobei der Messarm (40) radial an einer zur Drehachse (8) unterschiedlichen Schwenkachse (32) befestigt ist, die Messanordnung eine mechanische Kopplung zwischen dem Drehknopf (10) und der Schwenkachse (32) umfasst und eine zur Applanation des Auges benötigte Applanationskraft durch eine Drehung des Drehknopfes (10) um die Drehachse (8) erzeugbar ist, wobei die mechanische Kopplung Zugübertragungsmittel (12) umfasst, die einerseits via einen ersten Hebelarm (34) an der Schwenkachse (32) und andererseits via einen zweiten Hebelarm an der Drehachse (8) der Welle (6) befestigt sind, wobei die Zugübertragungsmittel (12) bei einer Drehung des Drehknopfes (10) um die Drehachse (8) insbesondere auf die Welle (6) aufwickelbar sind.

2. Vorrichtung nach Anspruch 1, wobei es zur Ausbalancierung der Messanordnung im Nullbereich wenigstens ein Ausgleichsgewicht (50, 52) umfasst.

3. Vorrichtung nach Anspruch 2, wobei das wenigstens eine Ausgleichsgewicht (50, 52) derart angeordnet und/oder dimensioniert ist, dass bei Abstandsänderungen des Auges im Nullbereich ein an der Schwenkachse (32) angreifendes Gesamtdrehmoment als Summe von an der Schwenkachse (32) angreifenden einzelnen Drehmomenten infolge einer Zugübertragung durch die Zugübertragungsmittel (12) sowie infolge von Gewichtsverlagerungen des wenigstens einen Ausgleichsgewichts (50,52), des Messarms (40) und des Messkörpers (44) im Wesentlichen konstant ist.

4. Vorrichtung nach Anspruch 2 oder 3, wobei ein erstes Ausgleichsgewicht (50) als Gegengewicht zum Messarm (40) an der Schwenkachse (32) und ein zweites Ausgleichsgewicht (52) als Gegengewicht zum Messkörper (44) an der Schwenkachse (32) befestigt ist.

5. Vorrichtung nach einem der Ansprüche 1-4, wobei die Zugübertragungsmittel eine Feder (18), insbesondere eine Schraubenfeder, umfassen, und mit welcher die Zugübertragungsmittel (12) an einem den ersten Hebelarm definierenden Hebel (34) befestigt sind.

6. Vorrichtung nach einem der Ansprüche 1-5, wobei die Zugübertragungsmittel ein Band (12) aus einem biegsamen und undehnbaren Material umfassen.

7. Vorrichtung nach Anspruch 6, wobei die Zugübertragungsmittel ein Stahlband (12) mit einer Dicke von weniger als 0.05 mm, vorzugsweise mit einer Dicke von 0.02 mm oder weniger umfassen.

8. Vorrichtung nach einem der Ansprüche 1-7, mit einer Anzeigevorrichtung (166), mit welcher der der Applanationskraft entsprechende Drehwinkel des Drehknopfes (10) optisch darstellbar ist, wobei die Anzeigevorrichtung (166) insbesondere ein Display umfasst, auf welchem die Applanationskraft anzeigbar ist.

9. Vorrichtung nach einem der Ansprüche 1-8, mit einem Mikroprozessor (170) und einem an der Welle (6) befestigten Winkelgeber (160) zur Umwandlung des eingestellten, der Applanationskraft entsprechenden Drehwinkels in ein elektrisches Signal, welches vom Mikroprozessor (170) in einen die Applanationskraft repräsentierenden, digitalen Wert umwandelbar ist, wobei der digitale Wert als Applanationskraft mit der Anzeigevorrichtung (166) optisch darstellbar ist, wobei der Winkelgeber (160) insbesondere linear ist.

10. Vorrichtung nach Anspruch 9, wobei es einen Sender (174) für eine kurzreichweitige Funkübertragungstechnik, insbesondere einen Bluetooth Sender, umfasst, mit welchem der digitale Wert an einen entsprechenden Empfänger übermittelbar ist.

11. Vorrichtung nach einem der Ansprüche 1-10, wobei es als Applanationstonometer nach Goldmann ausgebildet ist.

12. Verfahren zur Bestimmung eines Augeninnendruckes eines Auges, bei welchem das Auge mit einem Messkörper (44), welcher an einem radial an einer Schwenkachse (32) befestigten Messarm (40) befestigt ist, applaniert wird, wobei die zur Applanation benötigte Applanationskraft durch eine Drehung eines auf einer Welle (6) befestigten Drehknopfes (10) um eine Drehachse (8) erzeugt und mittels einer mechanischen Kopplung zwischen dem Drehknopf (10) und der zur Drehachse (8) unterschiedlichen Schwenkachse (32) auf den Messarm (40) übertragen wird, wobei die Applanationskraft mittels eines Zugübertragungsmittels (12) auf den Messarm (40) übertragen wird, indem die Zugübertragungsmittel (12) einerseits via einen ersten Hebelarm (34) an der Schwenkachse (32) und andererseits via einen zweiten Hebelarm an der Drehachse (8) der Welle (6) befestigt werden, wobei die Zugübertragungsmittel (12) bei einer Drehung des Drehknopfes (10) um die Drehachse (8) insbesondere auf die Welle (6) aufgewickelt werden.

## Claims

1. Device for determining the intraocular pressure of an eye having a measurement arrangement comprising a measurement body (44), attached to a measurement arm (40), for applanation of the eye and a rotary knob (10) which is attached to a shaft (6) and can rotate about a rotational axis (8) of the shaft (6), with the measurement arm (40) being attached radially to a pivot axis (32) which is different from the rotational axis (8), the measurement arrangement comprising a mechanical coupling between the rotary knob (10) and the pivot axis (32), and a rotation of the rotary knob (10) around the rotational axis (8) being able to generate an applanation force required for applanation of the eye, the mechanical coupling comprising tension transmission means (12) which are attached, on the one hand, to the pivot axis (32) via a first lever arm (34), and, on the other hand, to the rotational axis (8) of the shaft (6) via a second lever arm, with the tension transmission means (12) being able to be wound-up in particular onto the shaft (6) by a rotation of the rotary knob (10) about the rotational axis (8).

2. Device according to Claim 1, wherein the measurement arrangement comprises at least one balancing weight (50, 52) in the zero region for balancing the measurement arrangement.

3. Device according to Claim 2, wherein the at least one balancing weight (50, 52) is arranged and/or dimensioned in such a manner that in the case of changes in spacing of the eye in the zero region, an overall torque of the measurement arm (40) and the measurement body (44) acting on the pivot axis (32) is substantially constant, the overall torque being the sum of individual torques acting on the pivot axis (32) as a result of tension transmission by the tension transmission means (12) and as the result of weight shifts of the at least one balancing weight (50, 52).

4. Device according to Claim 2 or 3, wherein a first balancing weight (50) is attached to the pivot axis (32) as a counterweight to the measurement arm (40), and a second balancing weight (52) is attached to the pivot axis (32) as a counterweight to the measurement body (44).

5. Device according to one of Claims 1-4, wherein the tension transmission means comprise a spring (18), in particular a helical spring, by means of which the tension transmission means (12) are attached to a lever (34) defining the first lever arm.

6. Device according to one of Claims 1-5, wherein the tension transmission means comprise a band (12) consisting of a flexible and inductile material.

7. Device according to Claim 6, wherein the tension transmission means comprise a steel band (12) with a thickness of less than 0.05 mm, preferably with a thickness of 0.02 mm or less.

8. Device according to one of Claims 1-7, comprising a display device (166) by means of which the rotation angle of the rotary knob (10) corresponding to the applanation force can be displayed optically, wherein the display device (166) in particular comprises a display on which the applanation force can be displayed.

9. Device according to one of Claims 1-8, comprising a microprocessor (170) and an angle transmitter (160) attached to the shaft (6) for converting the set rotation angle corresponding to the applanation force into an electrical signal which can be converted into a digital value representing the applanation force by the microprocessor (170), wherein the digital value can be displayed optically as the applanation force by means of the display device (166), wherein the angle transmitter (160) is, in particular, linear.

10. Device according to Claim 9, wherein the device comprises a transmitter (174) for short-range radio-transmission technique, in particular a Bluetooth transmitter, by means of which the digital value can be transmitted to an appropriate receiver.

11. Device according to one of Claims 1-10, wherein the device is an applanation tonometer in the form of a Goldmann applanation tonometer.

12. Method for determining an intraocular pressure of an eye, in which the eye is applanate due to a measurement body (44) which is attached to a measurement arm (40) attached radially to a pivot axis (32), wherein the applanation force required for the applanation is generated by rotating a rotary knob (10), attached to a shaft (6), around a rotation axis (8) and is transmitted to the measurement arm (40) by means of a mechanical coupling between the rotary knob (10) and the pivot axis (32) which is different from the rotational axis (8), by means of a tension transmission means (12), the applanation force being transmitted onto the measurement arm (40) by the tension transmission means (12) being attached, on the one hand, to the pivot axis (32) via a first lever arm (34), and, on the other hand, to the rotational axis (8) of the shaft (6) via a second lever arm, with the tension transmission means (12) being wound-up in particular onto the shaft (6) by a rotation of the rotary knob (10) about the rotational axis (8).

## Revendications

1. Dispositif de détermination de la pression interne d'un oeil, le dispositif présentant un ensemble de mesure qui comporte :
un corps de mesure (44) fixé sur un bras de mesure (40) et assurant l'aplanissement de l'oeil ainsi qu'un bouton rotatif (10) fixé sur un arbre (6) et apte à tourner autour de l'axe de rotation (8) de l'arbre (6),
le bras de mesure (40) étant fixé radialement sur un axe de pivotement (32) différent de l'axe de rotation (8),
l'ensemble de mesure comportant un accouplement mécanique entre le bouton rotatif (10) et l'axe de pivotement (32),
la force d'aplanissement nécessaire pour l'aplanissement de l'oeil pouvant être exercée par une rotation du bouton rotatif (10) autour de l'axe de rotation (8),
l'accouplement mécanique comportant des moyens (12) de transfert de traction fixés d'une part par un premier bras de levier (34) sur l'axe de pivotement (32) et d'autre part par un deuxième bras de levier sur l'axe de rotation (8) de l'arbre (6),
les moyens (12) de transfert de traction pouvant être enroulés autour de l'axe de rotation (8) et en particulier autour de l'arbre (6) par une rotation du bouton rotatif (10).

2. Dispositif selon la revendication 1, qui comporte au moins un poids d'équilibrage (50, 52) qui assure l'équilibre de l'ensemble de mesure dans la plage du zéro.

3. Dispositif selon la revendication 2, dans lequel le ou les poids d'équilibrage (50, 52) sont disposés et/ou dimensionnés de telle sorte qu'en cas de modification de la distance de l'oeil dans la plage du zéro, le couple global de rotation qui agit sur l'axe de pivotement (32) et qui correspond à la somme des différents couples de rotation qui agissent sur l'axe de pivotement (32) suite à un transfert de traction par les moyens (12) de transfert de traction ainsi que suite aux déplacements du poids du ou des poids d'équilibrage (50, 52) du bras de mesure (40) du corps de mesure (44) soit essentiellement constant.

4. Dispositif selon les revendications 2 ou 3, dans lequel un premier poids d'équilibrage (50) qui sert de contrepoids au bras de mesure (40) est fixé sur l'axe de pivotement (32) et un deuxième poids d'équilibrage (52) qui sert de contrepoids au corps de mesure (44) est fixé sur l'axe de pivotement (32).

5. Dispositif selon l'une des revendications 1 à 4, dans lequel les moyens de transfert de traction comportent un ressort (18) et en particulier un ressort hélicoïdal par lequel les moyens (12) de transfert de traction sont fixés sur un levier (34) qui définit le premier bras de levier.

6. Dispositif selon l'une des revendications 1 à 5, dans lequel les moyens de transfert de traction comportent un ruban (12) en un matériau flexible et inextensible.

7. Dispositif selon la revendication 6, dans lequel les moyens de transfert de traction comportent un feuillard d'acier (12) d'une épaisseur inférieure à 0,05 mm et de préférence d'une épaisseur de 0,02 mm ou moins.

8. Dispositif selon l'une des revendications 1 à 7, doté d'un dispositif d'affichage (166) par lequel l'angle de rotation du bouton rotatif (10) qui correspond à la force d'aplanissement peut être présenté visuellement, le dispositif d'affichage (166) comportant en particulier un affichage sur lequel la force d'aplanissement peut être affichée.

9. Dispositif selon l'une des revendications 1 à 8, doté d'un microprocesseur (170) et d'un détecteur d'angle (160) fixé sur l'arbre (6) et permettant de convertir l'angle de rotation qui a été établi en correspondance à la force d'aplanissement en un signal électrique qui peut être converti par le microprocesseur (170) en une valeur numérique qui représente la force d'aplanissement, la valeur numérique représentant la force d'aplanissement pouvant être présentée visuellement par le dispositif d'affichage (166), le détecteur d'angle (160) étant en particulier linéaire.

10. Dispositif selon la revendication 9, qui comprend un émetteur (174) réalisé par la technologie de transmission radio à faible portée, en particulier un émetteur dit "bluetooth", par lequel la valeur numérique peut être transmise à un récepteur approprié.

11. Dispositif selon l'une des revendications 1 à 10, configuré comme tonomètre d'aplanissement selon Goldmann.

12. Procédé de détermination de la pression interne d'un oeil, dans lequel l'oeil est aplani à l'aide d'un corps de mesure (44) qui est fixé sur un bras de mesure (40) fixé radialement sur un axe de pivotement (32), la force d'aplanissement nécessaire pour l'aplanissement étant appliquée en faisant tourner autour d'un axe de rotation (8) un bouton rotatif (10) fixé sur un arbre (6) et étant transmise au bras de mesure (40) au moyen d'un accouplement mécanique entre le bouton rotatif (10) et l'axe de pivotement (32), différent de l'axe de rotation (8), la force d'aplanissement étant transférée sur le bras de mesure (40) au moyen d'un moyen (12) de transfert de traction par le fait que les moyens (12) de transfert de traction sont fixés d'une part par un premier bras de levier (34) sur l'axe de pivotement (32) et d'autre part par un deuxième bras de levier sur l'axe de rotation (8) de l'arbre (6), les moyens (12) de transfert de traction étant enroulés autour de l'axe de rotation (8) et en particulier sur l'arbre (6) lors de la rotation du bouton rotatif (10).
